(19) Europäisches Patentamt
European Patent Office
Office européen des brevets

(11) **EP 3 573 494 B1**

(12) **FASCICULE DE BREVET EUROPEEN**

(45) Date de publication et mention de la délivrance du brevet:
**13.10.2021 Bulletin 2021/41**

(21) Numéro de dépôt: **18702787.5**

(22) Date de dépôt: **24.01.2018**

(51) Int Cl.:
***A45D 40/08*** *(2006.01)*

(86) Numéro de dépôt international:
**PCT/FR2018/050165**

(87) Numéro de publication internationale:
**WO 2018/138436 (02.08.2018 Gazette 2018/31)**

(54) **DISPOSITIF DE CONDITIONNEMENT ET DE DISTRIBUTION D'UNE COMPOSITION COSMÉTIQUE**

VORRICHTUNG ZUM VERPACKEN UND AUSGEBEN EINER KOSMETISCHEN ZUSAMMENSETZUNG

DEVICE FOR PACKAGING AND DISPENSING A COSMETIC COMPOSITION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.01.2017 FR 1750749**

(43) Date de publication de la demande:
**04.12.2019 Bulletin 2019/49**

(73) Titulaire: **Chanel Parfums Beauté**
**92200 Neuilly-sur-Seine (FR)**

(72) Inventeurs:
• **LAUER, Alexandre**
**93694 Pantin Cedex (FR)**
• **PIRES LEITAO, Sylvie**
**93694 Pantin Cedex (FR)**
• **CASTEX, Nicolas**
**92521 Neuilly (FR)**

(74) Mandataire: **Plasseraud IP**
**66, rue de la Chaussée d'Antin**
**75440 Paris Cedex 09 (FR)**

(56) Documents cités:
EP-A1- 1 306 074     EP-A2- 1 072 214
WO-A1-00/19860     WO-A1-99/01052
FR-A- 674 267     FR-A1- 2 981 835

**Description**

La présente invention concerne un dispositif de conditionnement et de distribution d'une composition cosmétique selon la revendication 1.

EP1072214 divulgue une tête de distribution de produit semi-solide.

Les compositions cosmétiques selon l'invention peuvent notamment relever du domaine du maquillage, du soin, ou des compositions parfumantes. Entre autres produits de maquillage, on peut citer le rouge à lèvres, le fond de teint, le correcteur de teint, la base de teint, l'anti-cernes, le fard à joues ("blush"), l'illuminateur ("highlighter"), les ombres à paupière, et plus généralement tout produit de maquillage pour le corps. Les produits de soin peuvent être des baumes à lèvres, des soins hydratants, des soins anti-rides, des soins de protection solaire.

Les produits de consistance pâteuse à solide à température ambiante, destinés en particulier à l'application sur la peau, sont généralement conditionnés soit sous forme de stick - ou *raisin,* soit dans des réservoirs comme des pots ou des godets. Ces derniers nécessitent alors l'utilisation d'un applicateur, comme une éponge ou un pinceau, ce qui ne permet pas au consommateur d'emporter son produit et de l'utiliser en toutes circonstances.

Les préparations cosmétiques sous forme de stick, comme les rouges à lèvres, sont largement connues et sont utilisées régulièrement par de nombreux consommateurs dans les domaines les plus vastes du soin du corps. Les sticks sont désirables pour leur facilité d'application et leur portabilité.

Cependant, ils peuvent avoir pour inconvénient d'être perçus comme étant trop durs lorsqu'ils sont appliqués sur la peau. A l'inverse, ils peuvent perdre de leurs propriétés mécaniques et esthétiques sous l'effet de variations de températures par exemple. Ceci a pour effet de limiter leur attrait et leur résistance lors de l'application, pouvant conduire à un déchaussement ou à une casse du stick sous l'effet de la pression exercée par le consommateur lors de l'étalement du produit.

Afin d'utiliser de telles compositions cosmétiques, et en particulier lorsqu'il s'agit de les appliquer par exemple sur la peau ou les lèvres, il est important que ces compositions présentent de bonnes propriétés rhéologiques. En particulier, elles doivent être suffisamment fondantes pour être étalées facilement sur la peau sans compromettre les propriétés sensorielles ni le confort du consommateur, mais elles doivent également présenter de bonnes propriétés de résistance mécaniques pour éviter les risques de casse du stick. Ces contraintes peuvent s'avérer limitantes du point de vue de la formulation. En particulier, des compositions cosmétiques peuvent être chargées en pigments pour obtenir une couleur intense dans le domaine de maquillage. De même, dans le domaine du soin, il peut être souhaitable d'utiliser des corps gras solides ou pâteux.

D'autres aspects problématiques sont parfois le dosage du produit et la tendance indésirable de certaines préparations à former des résidus sur la surface d'application.

Un but de l'invention est donc de remédier aux déficiences des moyens de conditionnement et d'application des compositions cosmétiques existants et de proposer un dispositif de conditionnement et de distribution d'une composition cosmétique adapté à l'application d'une composition cosmétique présentant une certaine dureté, et ce, sans l'intervention d'un applicateur.

A cet effet, on prévoit selon l'invention un dispositif de conditionnement et de distribution d'une composition cosmétique selon la revendication 1.

La dureté de la composition cosmétique est évaluée à 20°C à l'aide d'un texturomètre TA-XT Plus Microstable System de la société Swantech.

Le dispositif selon l'invention permet de conditionner et de conserver la composition cosmétique dans son état initial qui peut être un état pâteux, solide, structuré et/ou cassant, tout en l'appliquant sous une forme destructurée et ductile. En effet, lorsque la composition cosmétique passe à travers la grille, ses propriétés rhéologiques sont modifiées et la composition passe d'un état structuré et parfois cassant à un état déstructuré et ductile, plus fluide, se traduisant au niveau physique par la réduction de sa dureté.

Ainsi, l'invention permet de fournir un moyen de conditionnement et de distribution de compositions ayant une dureté élevée, qui sont difficiles à étaler directement sur la peau à l'état natif.

L'invention permet également de fournir un moyen de conditionnement et de distribution des compositions cosmétiques ayant une trop grande consistance pour être délivrées par le biais d'un flacon-pompe, mais ne présentant pas assez de résistance mécanique pour pouvoir être conditionnée sous forme de stick.

L'invention permet donc de distribuer de manière satisfaisante de telles compositions sans contraintes particulières au niveau de leur formulation. Le dispositif selon l'invention améliore également l'étalement, tout en conservant la gestuelle d'un stick à lèvres conventionnel dans le cas d'une application labiale, et en procurant dans tous les cas une grande facilité d'application en raison du caractère transportable du dispositif selon l'invention.

De plus, la composition cosmétique peut être masquée dans le dispositif si celui-ci présente par exemple des parois opaques ou non complètement transparentes, ce qui permet de s'affranchir de l'aspect esthétique de la composition cosmétique.

Le dispositif selon l'invention est avantageusement agréable à utiliser et génère une sensation agréable au toucher comme une sensation de douceur. Cette sensation peut par exemple être due à la texture ramollie obtenue après le passage de la composition cosmétique à travers la grille.

Avantageusement, le dispositif selon l'invention permet de maitriser et de moduler la quantité de composition cosmétique à appliquer. En effet, seule la composition ayant été déstructurée par son passage à travers la grille est susceptible d'être appliqué. En jouant sur l'épaisseur du film appliqué, il est ainsi possible de faire varier la couvrance, dans le cas d'un produit de maquillage, ou, plus généralement, de maitriser la dose appliquée.

Dans le cas d'une composition cosmétique de maquillage, la brillance du film appliqué sur la peau peut être augmentée par le passage à travers la ou les grille(s) du dispositif selon l'invention, ce qui peut être très avantageux pour certaines applications.

Grilles

Le dispositif selon l'invention peut présenter une seule grille ou, avantageusement, au moins deux grilles se faisant suite suivant une direction de passage de la composition cosmétique à travers les grilles. Cela permet notamment d'obtenir un effet de guidage plus important de la composition cosmétique, chaque grille orientant un peu plus la composition cosmétique dans la direction d'application. Le fait d'utiliser plusieurs grilles permet une déstructuration plus importante et/ou facilitée de la composition cosmétique. En effet, les effets déstructurant de chaque grille se cumulent et la composition cosmétique subit une première déstructuration lors de son passage - ou de son *extrusion* - à travers la première grille, et une déstructuration supplémentaire lors de chaque passage ultérieur à travers chaque grille supplémentaire.

Les orifices de chaque grille peuvent être superposés en vis-à-vis, ou au contraire les orifices peuvent être décalés. Par exemple, deux grilles peuvent être agencées de sorte qu'un coin d'un orifice traversant de la première grille est situé en vis-à-vis du centre d'un orifice traversant de la seconde grille.

Les orifices traversants présentent une plus grande dimension comprise entre 40 et 475 $\mu$m. De telles dimensions permettent avantageusement au consommateur de ne pas fournir un effort trop important pour faire passer la composition cosmétique à travers la ou les grilles, tout en permettant une déstructuration satisfaisante.

Lorsqu'il y a plusieurs grilles, les orifices sont avantageusement de plus grande dimension décroissante dans le sens du trajet de la composition.

Les orifices peuvent être de toute forme, et en particulier de section circulaire, ovale, hexagonale, quadrilatérale - notamment rectangulaire - ou pentagonale.

La grille unique ou la grille la plus externe du dispositif elle-même est constituée d'un matériau métallique. Et la grille ou au moins une des grilles est tissée, par exemple par un tissage formé par des fils de chaîne et des fils de trame, les fils de chaîne ayant classiquement un diamètre différent de celui des fils de trame.

Par "première grille" on entend la première grille à travers laquelle est extrudée la composition cosmétique. Par "grille la plus externe" on entend la dernière grille à travers laquelle passe la composition cosmétique avant d'être appliquée.

Selon un mode de réalisation particulier, le dispositif comprend deux grilles dans lequel la première grille présente des orifices de plus grande dimension compris entre 160 et 475 $\mu$m, et la grille externe présente des orifices de plus grande dimension compris entre 42 et 125 $\mu$m.

Composition

La composition cosmétique conditionnée et distribuée par le dispositif selon l'invention peut se présenter sous une grande variété de forme. Elle peut notamment se présenter sous forme anhydre, ou sous la forme d'une émulsion simple ou d'une émulsion multiple.

Dans le cas d'une émulsion simple, il peut s'agir d'une émulsion huile-dans-eau ou eau-dans-huile. Cela signifie qu'il s'agit d'une suspension colloïdale de gouttelettes d'une solution hydrophile dans une solution hydrophobe, ou à l'inverse, d'une suspension colloïdale de gouttelettes d'une solution hydrophobe dans une solution hydrophile. De telles émulsions ne sont donc pas limitées à proprement parler à des gouttelettes d'eau dans de l'huile ou d'huile dans de l'eau.

Dans le cas d'une émulsion multiple, on a une suspension colloïdale d'une émulsion dans une autre solution que l'on peut qualifier de solution-matrice. Si l'émulsion est une émulsion huile-dans-eau, la solution matrice sera une solution hydrophobe, et au contraire, si l'émulsion est une émulsion eau-dans-huile, la solution-matrice sera une solution aqueuse.

Il est possible d'utiliser des émulsions d'ordre supérieur telles que des émulsions eau-dans-huile-dans-eau-dans-huile ou huile-dans-eau-dans-huile-dans-eau, etc.

De telles émulsions comprennent donc une ou plusieurs phases aqueuses et/ou une ou plusieurs phases grasses.

La taille des gouttelettes est conditionnée par les propriétés des solutions utilisées et est suffisamment petite pour éviter que les gouttelettes s'agrègent entre elles. L'homme du métier, connaissant les sciences des colloïdes, saura adapter les propriétés de l'émulsion à la formulation des différentes phases utilisées, et réciproquement saura adapter la formulation des différentes phases s'il souhaite obtenir une taille de gouttelettes particulièrement avantageuse.

La composition est de préférence conditionnée sous une forme choisie parmi une composition anhydre coulée, un gel aqueux et un gel huileux.

De préférence, la composition cosmétique présente au moins une phase grasse structurée par un agent structurant de la phase grasse. La ou les phases grasses de la composition cosmétique comprennent de préférence 0,5 à 90% en poids d'au moins un agent structurant de la phase grasse. Selon un mode réalisation avantageux, l'agent structurant de la phase grasse peut être choisis parmi les cires ou les gélifiants de la phase grasse tels que les argiles rendues hydrophobes ou les élastomères de silicone, des corps gras pâteux, et leurs mélanges.

*Composition - cires*

Par « cire », on entend un corps gras ayant une température de fusion supérieure à 30°C et généralement inférieure à 110°C, qui est liquide dans les conditions de préparation de la composition et présente à l'état solide une organisation cristalline anisotrope. Les cires selon l'invention peuvent être choisies parmi les cires polaires, les cires apolaires, ou leurs mélanges.

Par "cire apolaire" on entend une cire ne comprenant que des atomes d'hydrogène et de carbone.

Par "cire polaire" on entend une cire comprenant au moins un hétéroatome comme l'oxygène, l'azote, le silicium, ou le phosphore.

Les cires utilisables peuvent être des cires d'origine animale, végétale, minérale ou synthétique.

Parmi les cires utilisables dans la présente invention on peut citer par exemple les cires hydrocarbonées comprenant une chaine alkyl de 10 à 60 atomes de carbone. Ladite chaine peut être saturée ou insaturée, linéaire ou ramifiée. Par "cire hydrocarbonée", on entend une cire formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, ester, éther, acide carboxylique, amine et/ou amide. Parmi les cires hydrocarbonées utilisables dans le cadre de l'invention on peut notamment citer:

- les alcools gras comme par exemple l'alcool stéarylique, cétéarylique ou leurs mélanges,
- les acides gras,
- les esters d'acides gras incluant les triglycérides, les diglycérides et les monoglycérides,
- les alcools gras ethoxylés,
- les acides gras ethoxylés et leurs sels,
- les ethers d'acide gras.

Parmi les esters d'acide gras utilisables dans le cadre de l'invention, on peut citer par exemple le stearyl stéarate, le stearyl heptanoate, le stearyl caprylate, le stearyl behenate, le stéaryl octyldodécanol, le cétéaryl béhénate, le béhényl béhénate, l'éthyleneglycol distéarate, les esters de jojoba et leurs mélanges.

A titre illustratif des cires convenant à l'invention, on peut également citer la cire d'abeille, la cire de lanoline, et les cires d'insectes de Chine, la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, les cires microcristallines, les paraffines et l'ozokérite; les cires de polyéthylène, et leurs mélanges.

On peut également citer le mélange d'esters de jojoba, de polyglycérine-3, de cire de fleur d'acacacia decurrens et de cire de graines de tournesol, disponible sous la dénomination commerciale Acticire® auprès de la société GATTEFOSSE. D'autres exemples de cires apolaires appropriées sont vendus par la société Sasol sous la dénomination commerciale Sasol Wax® C80; par la société Jeen sous les dénominations commerciales Jeenate® 2H à 6H; et par la société New Phase sous les marques commerciales Performalene® 500 et Performa® V343; un autre exemple de cire apolaire appropriée consiste en un mélange de polyéthylène linéaire de haut poids moléculaire et de copolymère éthylène / propylène disponible sous la marque déposée Lipwax® PZ80-20 de la société SAFIC-ALCAN.

Les cires selon l'invention peuvent aussi être choisies parmi les cires micronisées.

Les cires selon l'invention peuvent également être choisies parmi les cires de silicone, telles que les C30-C45 alkyl dimethicone, ou les cires fluorées.

*Composition - gélifiants de la phase grasse*

Comme indiqué précédemment, la composition selon l'invention peut comprendre au moins un gélifiant de la phase grasse.

Parmi les gélifiants de la phase grasse utilisables dans le cadre de l'invention, on peut notamment citer les élastomères d'organopolysiloxane (également appelé élastomère de silicone) qui peuvent par exemple se présenter sous forme de particules véhiculées dans au moins une huile non volatile siliconée ou hydrocarbonée, formant ainsi un gel. Il s'agit plus particulièrement d'un élastomère de silicone réticulé. L'élastomère présent dans la composition selon l'invention peut être choisi parmi les élastomères non émulsionnants ou émulsionnants.

Par " élastomère d'organopolysiloxane émulsionnant ", on entend un élastomère d'organopolysiloxane comprenant au moins une chaîne hydrophile, tels que les élastomères d'organopolysiloxane polyoxyalkylénés (polyoxyéthylénés, polyoxypropylénés) et les élastomères de silicone polyglycérolés. Comme élastomères d'organopolysiloxane polyoxyalkylénés, on peut utiliser ceux commercialisés sous les dénominations "KSG-21", "KSG-20", "KSG-30", "KSG-31", "KSG-33", "KSG-210", "KSG-310", "KSG-330", "KSG-340" par la société Shin Etsu, "DC9010", "DC9011" par la société Dow Corning. Comme élastomères d'organopolysiloxane polyglycérolés, on peut utiliser ceux vendus sous les dénominations " KSG-710 ", " KSG-810 ", " KSG-820 ", " KSG-830 ", " KSG-840 " par la société Shin Etsu.

Comme élastomères non-émulsionnants, on peut par exemple utiliser ceux vendus sous les dénominations "DC 9040", "DC 9041", "DC 9509", "DC 9505" par la société Dow Corning; "KSG-6", "KSG-15", " KSG-16", " KSG-18", "KSG-41", "KSG-42", "KSG-43", "KSG-44", par la société Shin Etsu ; Gransil SR 5CYC gel, Gransil SR DMF 10 gel, Gransil SR DC556 gel de la société Gransil RPS de Grant Industries ; 1229-02-167, 1229-02-168 et " SFE 839 " de la société General Electric.

Les gélifiants de la phase grasse utilisables dans la composition selon l'invention peuvent être des argiles modifiées afin de les rendre lipophiles. On peut citer les argiles montmorillonites modifiées hydrophobes comme les bentonites ou hectorites modifiées hydrophobes. On peut citer par exemple le produit Stearalkonium Bentonite (nom INCI) (produit de réaction de la bentonite et de l'ammonium quaternaire chlorure de stéaralkonium) tel que le produit commercial vendu sous le nom TIXOGEL MP 250 par la société Sud Chemie Rheologicals, United Catalysts Inc ou le produit Disteardimonium Hectorite (nom INCI) (produit de réaction de l'hectorite et du chlorure de distéaryldimonium) vendu sous le nom de Bentone 38 ou Bentone Gel par la société Elementis Specialities.

*Composition - corps gras pâteux*

La composition selon l'invention peut comprendre également au moins un composé pâteux à 25°C et pression atmosphérique. Par "pâteux " au sens de la présente invention, on entend un composé à changement d'état solide/liquide réversible, présentant à l'état solide une organisation cristalline anisotrope, et comportant à la température de 23°C une fraction liquide et une fraction solide.

Le composé pâteux peut en particulier être choisi parmi les composés pâteux synthétiques et les corps gras d'origine végétale.

Le ou les composés pâteux peuvent être en particulier choisis parmi :

- la lanoline et ses dérivés, tels que l'alcool de lanoline, les lanolines oxyéthylénées, la lanoline acétylée, les esters de lanoline tels que le lanolate d'isopropyle, les lanolines oxypropylénées ;
- la vaseline (également appelée petrolatum),
- les éthers de polyol choisis parmi les éthers de pentaérythritol et de polyalkylène glycol en C2-C4, les éthers d'alcool gras et de sucre, et leurs mélanges.
- les beurres d'origine végétale comme le beurre de mangue, tel que celui commercialisé sous la référence Lipex 203 par la société AARHUSKARLSHAMN, le beurre de karité, en particulier celui dont le nom INCI est Butyrospermum Parkii Butter, tel que celui commercialisé sous la référence Sheasoft((R)) par la société AARHUSKARLSHAMN, le beurre de cupuacu (Rain forest RF3410 de la société Beraca Sabara), le beurre de murumuru (RAIN FOREST RF3710 de la société Beraca Sabara), le beurre de cacao ; ainsi que la cire d'orange comme, par exemple, celle qui est commercalisée sous la référence Orange Péel Wax par la société Koster Keunen,
- les esters de phytostérol,
- les triglycérides d'acides gras et leurs dérivés,
- les esters de pentaérythritol,
- les huiles végétales totalement ou partiellement hydrogénées, comme par exemple l'huile de soja hydrogénée, l'huile de coprah hydrogénée, l'huile de colza hydrogénée, les mélanges d'huiles végétales hydrogénées tels que le mélange d'huile végétale hydrogénée de soja, coprah, palme et colza, par exemple le mélange commercialisé sous la référence Akogel((R)) par la société AARHUSKARLSHAMN (nom INCI Hydrogenated Vegetable Oil), l'huile de jojoba partiellement hydrogénée isomérisée trans fabriquée ou commercialisée par la société Désert Whale sous la référence commerciale Iso-Jojoba-50(R), l'huile d'olive partiellement hydrogénée comme, par exemple, le composé commercialisé sous la référence Beurrolive par la société Soliance,
- les esters d'huile de ricin hydrogénée, comme l'huile de ricin hydrogénée dimère dilinoléate par exemple le RISOCAST-DA-L vendu par KOKYU ALCOHOL KOGYO, l'isostéarate d'huile de ricin hydrogénée par exemple le SALACOS HCIS (V-L) vendu par NISSHIN OIL,

- les composés siliconés polymères ou non,
- les composés fluorés polymères ou non,
- et leurs mélanges.

*Composition - huiles*

En outre, la composition comprend de préférence 0,01 à 80% en poids par rapport au poids total de la composition, plus préférentiellement entre 0,5 et 50%, encore plus préférentiellement entre 1 et 30% d'huiles.

Les huiles utilisables peuvent être choisies de préférence parmi des huiles hydrocarbonées ou siliconées ou leurs mélanges.

Au sens de la présente invention, on entend par « huile » un composé liquide à température ambiante (25°C) et pression atmosphérique, et qui, lorsqu'il est introduit à raison d'au moins 1% en poids dans l'eau à 25°C, n'est pas du tout soluble dans l'eau, ou soluble à hauteur de moins de 10% en poids, par rapport au poids d'huile introduit dans l'eau. Les huiles utilisables dans la présente invention peuvent être volatiles ou non-volatiles. Les huiles peuvent être d'origine végétale, minérale ou synthétique.

*Huiles non volatiles*

Les huiles d'origine végétale comprennent notamment l'huile de ricin, l'huile d'amande douce, l'huile de tournesol, l'huile de son de riz, l'huile de noix de macadamia, l'huile d'olive, l'huile de germe de blé, l'huile d'arachide, l'huile d'argousier, l'huile de bourrache, l'huile d'onagre, l'huile de palme.

Les huiles d'origine minérale comprennent notamment la paraffine liquide, l'isoparaffine.

Les huile d'origine synthétique comprennent notamment les huiles hydrocarbonées telles que les (poly)ethers ou (poly)esters, en particulier les (poly)esters d'acides gras en C6 à C20 et d'alcools gras en C6 à C20 qui peuvent être avantageusement branchés tels que diisostéaryl malate, le dicaprylyl carbonate, le isononyl isononanoate; les huiles végétales ou leurs dérivés tels que l'huile de ricin hydrogénée; les acides gras branchés et/ou insaturés; les alcools gras branchés et/ou insaturés; les huiles de silicone telles que les polydimethylsiloxanes linéaires (nom INCI: Dimethicone) qui peuvent être optionnellement phénylées, les polydimethylsiloxanes cycliques; et leurs mélanges.

Lorsque la composition est destinée au maquillage ou au soin des lèvres, elle peut avantageusement comprendre au moins une huile brillante. Par "huile brillante" on entend une huile liquide à 25°C et pression atmosphérique caractérisée par un indice de réfraction supérieur ou égal à 1,47. Parmi les huiles brillantes utilisables selon l'invention, on peut citer:

- les huiles hydrocarbonées telles que le squalane, le polybutene, le polyisobutene hydrogéné, ou le polydecene hydrogéné,
- les huiles siliconées phénylées telles que celles identifiables par les noms INCI "phenyl trimethicone", phenylpropyldimethylsiloxysilicate" ou "trimethyl pentaphenyl trisiloxane",
- les huiles de silicone fluorées telles que celles identifiables par les noms INCI "perfluorononyl dimethicone,
- les amino ceramides telles que celles identifiables par les noms INCI "phytosteryl/octyldodecyl lauroyl glutamate" vendu notamment par la société Ajinomoto sous le nom commercial Eldew® PS203,
- et leurs mélanges.

*Huiles volatiles*

La composition selon l'invention peut également comprendre au moins une huile volatile à température ambiante. Par "huile volatile", on entend au sens de l'invention une huile susceptible de s'évaporer au contact de la peau ou de la fibre kératinique en moins d'une heure. Les huiles volatiles de l'invention sont des huiles cosmétiques volatiles, liquides à température ambiante, ayant une pression de vapeur non nulle, à température ambiante et pression atmosphérique.

Des exemples d'huiles volatiles appropriées comprennent des alcanes linéaires volatils tels que ceux décrits dans le document FR2933865 qui est incorporé ici à titre de référence. Des exemples d'alcanes linéaires volatils appropriés sont les alcanes en C9-C17, en particulier C10-C14, tels que le mélange d'undécane et de tridécane, disponible sous la dénomination commerciale Cetiol® Ultimate de BASF ou les alcanes en C15-C19 disponibles sous la dénomination commerciale Emogreen ® L15 de SEPPIC, ou alcanes en C12-14 disponibles sous le nom commercial Vegelight® 1214 LC de Biosynthis.

Il est clairement entendu que la composition selon l'invention peut comprendre des mélanges des huiles mentionnées ci-dessus.

*Composition - filmogènes*

La composition selon l'invention peut également comprendre au moins un polymère filmogène, en particulier susceptible d'apporter de la tenue et/ou des propriétés de non -transfert à la composition.

La composition selon l'invention peut en outre comprendre au moins un filmogène choisi parmi les filmogènes hydrocarbonés et/ou siliconés.

Un tel filmogène est généralement un polymère. Ce polymère filmogène peut être un polymère siliconé éventuellement modifié uréthane ou fluoré ou acrylate tel que les silicones (méth)acrylates commercialisées par JÉEN sous la dénomination Jéesil PS (qui inclus PS-VH, PS-VHLV, PS-CM, PS- CMLV et PS-DMLV),ou les polymères commercialisés par SHIN-ETSU sous les dénominations commerciales KP-545, KP-561 et KP-562, ou les polymères commercialisés par la société DOW CORNING sous les dénominations commerciales Dow Corning® FA 4003 DM, Dow Corning® FA 4002 ID et Dow Corning® FA 4001 CM. D'autres exemples de polymères filmogènes sont les résines de silicone et en particulier les résines MQ telles que les triméthylsiloxysilicates et les résines MT telles que les dérivés de silsesquioxane et notamment les polyméthylsilsesquioxanes, commercialisés notamment par la société SHIN-ETSU, ainsi que le polypropylsilsesquioxane commercialisé par la société DOW CORNING sous la dénomination commerciale Dow Corning® 670 ou le phénylpropyl polysilsesquioxane commercialisé par la société WACKER sous la dénomination commerciale BELSIL SPR45VP. Un autre exemple est constitué des polymères fluorosiliconés identifiés par le nom INCI trifluoropropyldiméthylsiloxy triéthylsiloxysilicate tels que celui commercialisé par la société GENERAL ELECTRIC sous la dénomination commerciale XS66-B8226. On peut également utiliser comme polymères filmogènes des polymères bioadhésifs obtenus par exemple par polycondensation de diméthiconol et de résine silicate MQ dans un solvant tel que l'heptane, qui sont notamment commercialisés par la société DOW CORNING sous les dénominations commerciales Dow Corning® BIO-PSA 7-4560 SILICONE ADHESIVE, Dow Corning® 7-4405 low tack et Dow Corning® 7-4505 high tack. D'autres exemples de polymères filmogènes sont les polyoléfines cycliques telles que le polycyclopentadiène, notamment commercialisé par la société KOBO sous la dénomination commerciale KOBOGUARD 5400, ou encore le polydicyclopentadiène. D'autres exemples encore de filmogènes sont constitués de copolymères de vinylpyrrolidone (VP) et/ou d'oléfines linéaires tels que les copolymères VP/hexadécène et VP/eicosène dont l'ANTARON V216 et l'ANTARON V220 de la société ISP ou encore les copolymères éthylène / acétate de vinyle tels que l'AC 400 de la société BAERLOCHER. D'autres polymères filmogènes susceptibles d'être utilisés dans cette invention sont des polyacrylates tels que le poly(acrylate d'éthyle) commercialisé notamment par la société CREATIONS COULEURS sous la dénomination commerciale CREASIL 7 ID.

*Composition - filtres ultraviolets*

La composition cosmétique peut également comprendre 0 à 50 % en poids d'au moins un filtre ultraviolet (UV), de préférence de 0 à 30%, plus préférentiellement de 0 à 20% en poids total de la composition. En particulier, la composition selon l'invention peut comprendre au moins un filtre solaire organique ou minéral ou un mélange des deux.

A titre d'illustration des filtres UV organiques et de façon non limitative, on peut citer :

- les anthranilates, en particulier l'anthranilate de menthyle ;
- les benzophénones, en particulier la benzophénone-1, la benzophénone-3 ou oxybenzone, la benzophénone-5, la benzophénone-6, la benzophénone-8, la benzophénone-9, la benzophénone-12, et préférentiellement la Benzophénone-2 (Oxybenzone), ou la Benzophénone-4 (Uvinul MS40® disponible chez B.A.S.F.) ;
- les benzylidènes-camphres, en particulier le 3-benzylidène-camphre, l'acide benzylidènecampho-sulfonique, le benzalkoniumméthosulfate de Camphre, le polyacrylamidométhylbenzylidène camphre, l'acide téréphthalylidène dicamphre sulfonique, et préférentiellement le 4-méthylbenzylidène camphre (Eusolex 6300® disponible chez Merck) ;
- les benzimidazoles, en particulier le benzimidazilate (Neo Heliopan AP® disponible chez Haarmann et Reimer), ou l'acide phénylbenzimidazole sulfonique (PARSOL HS® disponible chez DSM) ;
- les benzotriazoles, en particulier le drométrizole trisiloxane, ou le méthylène bis-benzotriazolyltétraméthylbutylphénol (Tinosorb M® disponible chez Ciba) ;
- les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, Kaempferia galanga root extract (TEGO GALANGA d'EVONIK contenant 98% ethyl-p-methoxycinnamate) et préférentiellement l'octylméthoxycinnamate (Parsol MCX® disponible chez Hoffmann La Roche) ;
- les diphenylacrylates en particulier l'éthocrylène (Uvinul N35® disponible chez B.A.S.F.), ou l'octocrylène (Uvinul 539® disponible chez B.A.S.F.) ou Ethylhexyl methoxycrylene (SOLASTAY disponible chez HALLSTAR) ;
- les dibenzoylméthanes, en particulier le butyl méthoxydibenzoylméthane (Parsol 1789®) ; les imidazolines, en particulier l'éthylhexyl diméthoxybenzylidène dioxoimidazoline;
- les PABA, en particulier l'éthyl Dihydroxypropyl PABA, l'éthylhexyldiméthyl PABA, le glycéryl PABA, le PABA, le PEG-25 PABA, ou l'éthyl PABA (benzocaïne) ;

- les triazines, en particulier l'anisotriazine (Tinosorb S® disponible chez Ciba) ou la diéthylhexylbutamido-triazone (Uvasorb HEB® disponible chez 3V Sigma), l'éthylhexyltriazone (Uvinul T150® disponible chez B.A.S.F.), le Tris-Biphenyl Triazine (Tinosorb 2AB disponible chez BASF) ;
- les benzoates, en particulier le N-hexyl 2-(4-diethylamino-2-hydroxybenzoyl)benzoate (Uvinul A+ disponible chez BASF) ou en mélange avec octyl methoxycinnamate (Uvinul A+B disponible chez BASF) ;
- les benzalmalonates, en particulier le Polysilicone-15 (Parsol SLX disponible chez DSM) :
- les benzoxazoles en particulier le 2,4-Bis[4-[5-(1,1-dimethylpropyl)benzoxazol-2-yl]phenylimino]-6-[(2-ethylhexyl)imino]- 1,3,5-triazine, (Uvasorb K2A disponible chez Sigma 3V) ;
- les salicylates, en particulier le salicyclate de dipropylèneglycol, le salicylate d'éthylhexyle, l'homosalate, le butyloctyl Salicylate (HALLBRITE BHB disponible chez HALLSTAR ou le TEA salicylate.

Les filtres UV inorganiques utilisés sont par exemple des particules d'oxyde métallique ayant une taille moyenne de particule élémentaire inférieure ou égale à 300 nm, de préférence inférieure ou égale à 100 nm.

Ils peuvent être notamment choisis parmi les oxydes de titane, de zinc, de fer, de zirconium, de cérium ou leurs mélanges. Les oxydes de titane peuvent se présenter sous une forme cristallisée de type rutile et/ou anatase, et/ou sous une forme amorphe ou substantiellement amorphe.

Les pigments d'oxydes de titane enrobés peuvent être enrobés:

- de silice (SUNVEIL d'IKEDA) ;
- de silice et d'oxyde de fer (SUNVEIL F d'IKEDA) ;
- de silice et de polyglyceryl-10 stearate (COSMESERVE WP-40W de IWASE COSFA) ;
- de silice et d'alumine (MICROTITANIUM DIOXIDE MT500 SA et MICROTITANIUM DIOXIDE MT 100 SA de TAYCA, TIOVEIL de TIOXIDE) ;
- d'alumine (TIPAQUE TTO-55 (B) et TIPAQUE TTO-55 (A) d'ISHIHARA, et UVT 14/4 de KEMIRA) ;
- de TiO2 rutile traité avec l'alumine et de la silice enrobé de glycérol (UV TITAN M212 de KEMIRA) ;
- de TiO2 rutile traité avec l'alumine et de la diméthicone (UV TITAN M195 de KEMIRA) ;
- d'alumine et de stéarate d'aluminium (MICROTITANIUM DIOXIDE MT 100 T, MT 100 TV, MT 100 TX, MT 100 Z, MT-01 de TAYCA, Solaveil CT-10 W et Solaveil CT 100 de UNIQEMA et Eusolex T-AVO de MERCK) ;
- de silice, d'alumine et d'acide alginique (MT-100 AQ de TAYCA) ;
- d'alumine et de laurate d'aluminium (MICROTITANIUM DIOXIDE MT 100 S de TAYCA) ;
- d'alumine, de methicone et d'acide polyhydroxystearique (INP60T7 de KOBO) ;
- d'oxyde de fer et de stéarate de fer (MICROTITANIUM DIOXIDE MT 100 F de TAYCA) ;
- d'oxyde de zinc et de stéarate de zinc (BR 351 de TAYCA) ;
- de silice et d'alumine et traités par une silicone (MICROTITANIUM DIOXIDE MT 600 SAS, MICROTITANIUM DIOXIDE MT 500 SAS ou MICROTITANIUM DIOXIDE MT 100 SAS de TAYCA) ;
- de silice, d'alumine, de stéarate d'aluminium et traités par une silicone (STT-30-DS de TITAN KOGYO) ;
- d'alumine et traités par une silicone (TIPAQUE TTO-55 (S) de ISHIHARA, ou UV TITAN M 262 de KEMIRA) ;
- de triéthanolamine (STT-65-S de TITAN KOGYO) ;
- d'acide stéarique (TIPAQUE TTO-55 (C) de ISHIHARA ;
- d'hexamétaphosphate de sodium (MICROTITANIUM DIOXIDE MT 150 W de TAYCA) ;
- de TiO2 traité par l'octyl triméthyl silane (T 805 par la société DEGUSSA SILICES) ;
- de TiO2 traité par un polydiméthylsiloxane (70250 Cardre UF TiO2S13 par CARDRE) ;
- de TiO2 anatase/rutile traité par un polydiméthylhydrogénosiloxane (MICRO TITANIUM DIOXYDE USP GRADE HYDROPHOBIC par COLOR TECHNIQUES) ;
- de TiO2 rutile traité par l'alumine, l'acide stéarique (UV TITAN M160 par KEMIRA) ;
- de TiO2 traité par l'alumine hydroxyde, l'acide stéarique et le triethoxycaprylylsilane (ALT-T-400 par MAPRECOS ou Titanium Dioxide & Aluminium hydroxide & Stearic acid (ST-705SA /TITAN KOGYO)) ;
- de TiO2 dopé au manganese (OPT1-PW de Croda).

Les pigments d'oxyde de titane non enrobés sont par exemple :

- MICROTITANIUM DIOXIDE MT 500 B ou MICROTITANIUM DIOXIDE MT600 B par la société TAYCA ;
- P 25 par la société DEGUSSA ;
- Oxyde de titane transparent PW par la société WACKHER ;
- UFTR par la société MIYOSHI KASEI ;
- ITS par la société TOMEN ;

et TIOVEIL AQ par la société TIOXIDE.

Les pigments d'oxyde de zinc non enrobés, sont par exemple:

- Z-cote par la société Sunsmart;
- Nanox par la société Elementis;
- Nanogard WCD 2025 par la société Nanophase Technologies.

Les pigments d'oxyde de zinc enrobés sont par exemple:

- Oxide zinc CS-5 par la société Toshibi (ZnO enrobé par polymethylhydrogenesiloxane);
- Nanogard Zinc Oxide FN par la société Nanophase Technologies (en dispersion à 40% dans le Finsolv TN, benzoate d'alcools en C12-C15);
- DAITOPERSION ZN-30 et DAITOPERSION Zn-50 par la société Daito (dispersions dans cyclopolyméthylsiloxane /polydiméthylsiloxane oxyéthyléné, contenant 30% ou 50% de nano-oxydes de zinc enrobés par la silice et le s polyméthylhydrogènesiloxane);
- NFD Ultrafine ZnO par la société Daikin (ZnO enrobé par phosphate de perfluoroalkyle et copolymère à base de perfluoroalkyléthyle en dispersion dans du cyclopentasiloxane);
- SPD-Z1 par la société Shin-Etsu (ZnO enrobé par polymère acrylique greffé silicone, dispersé dans cyclodiméthylsiloxane);
- Escalol Z100 par la société ISP (ZnO traité alumine et dispersé dans le mélange methoxycinnamate d'ethylhexyle / copolymère PVP-hexadecene / methicone);
- Fuji ZnO-SMS-10 par la société Fuji Pigment (ZnO enrobé silice et polymethylsilsesquioxane) ;
- Nanox Gel TN par la société Elementis (ZnO dispersé à 55% dans du benzoate d'alcools en C12-C15 avec polycondensat d'acide hydroxystéarique) ;
- OTS-5 MZ-500 par la société DAITO (ZnO dispersé dans triethoxycaprylylsilane).

Les pigments d'oxyde de cérium non enrobés peuvent être par exemple ceux vendus sous la dénomination COLLOIDAL CERIUM OXIDE par la société RHONE POULENC.

*Composition - pigments*

La composition selon l'invention peut comprendre de 0 à 50% en poids d'au moins un pigment.

Par "pigments", il faut comprendre des particules blanches ou colorées, minérales et/ou organiques, insolubles dans une solution aqueuse, destinées à colorer et/ou opacifier la composition et/ou le dépôt réalisé à partir de la composition.

Les pigments peuvent être choisis parmi les pigments minéraux, les laques organiques, les nacres, les pigments à effet optiques, comme les particules réfléchissantes ou les pigments interférentiels.

Les pigments minéraux peuvent être choisis parmi les pigments d'oxyde métallique, les oxydes de chrome, les oxydes de fer, le dioxyde de titane, les oxydes de zinc, les oxydes de cérium, les oxydes de zirconium, le violet de manganèse, le bleu de prusse, le bleu outremer, le bleu ferrique, le bleu outremer, l'hydrate de chrome et leurs mélanges.

La composition selon l'invention peut également comprendre un oxyde de titane pigmentaire particulier enrobé par de l'hydroxyde d'aluminium et de l'acide stéarique commercialisé par la société Titan Kogyo sous le nom ST 705 SA. Ce pigment présente une forte transmittance des rayons de couleur rouge tout en bloquant les autres rayons du spectre visible, et augmente le facteur de protection solaire.

La composition cosmétique peut également comprendre 0 à 50%, de préférence 0 à 30% en poids par rapport au poids total de la composition d'au moins un pigment interférentiel. Les pigments interférentiels sont des pigments aptes à générer de la couleur par un phénomène d'interférence. Ils peuvent être constitués d'un substrat, par exemple le mica naturel, la silice, le mica synthétique, le dioxyde de titane ou le verre, autour duquel sont déposées une ou plusieurs couches d'un matériau d'indice de réfraction différent comme le dioxyde de titane, l'oxyde de fer ou la silice par exemple.

A titre illustratif des pigments interférentiels pouvant être introduits dans la composition, on peut citer les pigments interférentiels de couleur or notamment commercialisés par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les pigments interférentiels bronzes notamment commercialisés par la société MERCK sous la dénomination Bronze fine (17384) (Colorona) et Bronze (17353) (Colorona) et par la société ENGELHARD sous la dénomination Super bronze (Cloisonne) ; les pigments interférentiels oranges notamment commercialisés par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les pigments interférentiels de teinte brune notamment commercialisés par la société ENGELHARD sous la dénomination Nu-antique copper 340XB (Cloisonne) et Brown CL4509 (Chromalite) ; les pigments interférentiels à reflet cuivre notamment commercialisés par la société ENGELHARD sous la dénomination Copper 340A (Timica) ; les pigments interférentiels à reflet rouge notamment commercialisés par la société MERCK sous la dénomination Sienna fine (17386) (Colorona) ; les pigments interférentiels à reflet jaune notamment commercialisés par la société ENGELHARD sous la dénomination Yellow (4502) (Chromalite) ; les pigments interférentiels de teinte rouge à reflet or notamment commercialisés par la société ENGELHARD sous la dénomination Sunstone G012 (Gemtone) ; les pigments interférentiels roses notamment commercialisés par la société ENGELHARD sous la dénomination Tan opale G005 (Gemtone) ; les pigments interférentiels noires à reflet or notamment commercialisés par la société ENGELHARD sous la dénomination Nu antique bronze 240 AB (Timica), les pigments interférentiels bleus notamment commercialisés par la société MERCK sous la dénomination Matte blue (17433) (Microna), les pigments interférentiels blancs à reflet argenté notamment commercialisés par la société MERCK sous la dénomination Xirona Silver et les pigments interférentiels orangés rosés vert doré notamment commercialisés par la société MERCK sous la dénomination Indian summer (Xirona) et leurs mélanges.

Les laques organiques sont des pigments organiques formés d'un colorant fixé sur un substrat. Elles peuvent être par exemple choisies parmi :

- le carmin de cochenille ;
- les pigments organiques de colorants azoïques, anthraquinoniques, indigoïdes, xanthéniques, pyréniques, quinoliniques, de triphénylméthane, de fluorane. Parmi les pigments organiques, on peut notamment citer ceux connus sous les dénominations suivantes : D&C Blue n.deg. 4, D&C Brown n.deg. 1, D&C Gréen n.deg. 5, D&C Gréen n.deg. 6, D&C Orange n.deg. 4, D&C Orange n.deg. 5, D&C Orange n.deg.10, D&C Orange n.deg. 1 1, D&C Red n.deg. 6, D&C Red n.deg. 7, D&C Red n.deg. 17, D&C Red n.deg.21, D&C Red n.deg. 22, D&C Red n.deg. 27, D&C Red n.deg. 28, D&C Red n.deg. 30, D&C Red n.deg. 31, D&C Red n.deg. 33, D&C Red n.deg. 34, D&C Red n.deg. 36, D&C Violet n.deg. 2, D&C Yellow n.deg. 7, D&C Yellow n.deg. 8, D&C Yellow n.deg. 10, D&C Yellow n.deg. 1 1, FD&C Blue n.deg. 1, FD&C Gréen n.deg. 3, FD&C Red n.deg. 40, FD&C Yellow n.deg. 5, FD&C Yellow n.deg. 6.
- les sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Les laques organiques peuvent également être supportées par un support organique tel que la colophane ou le benzoate d'aluminium, par exemple.

Parmi les laques organiques, on peut en particulier citer celles connues sous les dénominations suivantes : D&C Red n.deg. 2 Aluminium lake, D&C Red n.deg. 3 Aluminium lake, D&C Red n.deg. 4 Aluminium lake, D&C Red n.deg. 6 Aluminium lake, D&C Red n.deg. 6 Barium lake, D&C Red n.deg. 6 Barium/Strontium lake, D&C Red n.deg. 6 Strontium lake, D&C Red n.deg. 6 Potassium lake, D&C Red n.deg. 7 Aluminium lake, D&C Red n.deg. 7 Barium lake, D&C Red n.deg. 7 Calcium lake, D&C Red n.deg. 7 Calcium/Strontium lake, D&C Red n.deg. 7 Zirconium lake, D&C Red n.deg. 8 Sodium lake, D&C Red n.deg. 9 Aluminium lake, D&C Red n.deg. 9 Barium lake, D&C Red n.deg. 9 Barium/Strontium lake, D&C Red n.deg. 9 Zirconium lake, D&C Red n.deg. 10 Sodium lake, D&C Red n.deg. 19 Aluminium lake, D&C Red n.deg. 19 Barium lake, D&C Red n.deg. 19 Zirconium lake, D&C Red n.deg. 21 Aluminium lake, D&C Red n.deg. 21 Zirconium lake, D&C Red n.deg. 22 Aluminium lake, D&C Red n.deg. 27 Aluminium lake, D&C Red n.deg. 27 Aluminium/Titanium/Zirconium lake, D&C Red n.deg. 27 Barium lake, D&C Red n.deg. 27 Calcium lake, D&C Red n.deg. 27 Zirconium lake, D&C Red n.deg. 28 Aluminium lake, D&C Red n.deg. 30 lake, D&C Red n.deg. 31 Calcium lake, D&C Red n.deg. 33 Aluminium lake, D&C Red n.deg. 34 Calcium lake, D&C Red n.deg. 36 lake, D&C Red n.deg. 40 Aluminium lake, D&C Blue n.deg. 1 Aluminium lake, D&C Gréen n.deg. 3 Aluminium lake, D&C Orange n.deg. 4 Aluminium lake, D&C Orange n.deg. 5 Aluminium lake, D&C Orange n.deg. 5 Zirconium lake, D&C Orange n.deg. 10 Aluminium lake, D&C Orange n.deg. 17 Barium lake, D&C Yellow n.deg. 5 Aluminium lake, D&C Yellow n.deg. 5 Zirconium lake, D&C Yellow n.deg. 6 Aluminium lake, D&C Yellow n.deg. 7 Zirconium lake, D&C Yellow n.deg. 10 Aluminium lake, FD&C Blue n.deg. 1 Aluminium lake, FD&C Red n.deg. 4 Aluminium lake, FD&C Red n.deg. 40 Aluminium lake, FD&C Yellow n.deg. 5 Aluminium lake, FD&C Yellow n.deg. 6 Aluminium lake.

On peut également citer les colorants liposolubles tels que par exemple le rouge Soudan, le DC Red 17, le DC Gréen 6, le .beta.-carotène, l'huile de soja, le brun Soudan, le DC Yellow 1 1, le DC Violet 2, le DC orange 5, le jaune quinoléine.

Les matériaux chimiques correspondant à chacune des matières colorantes organiques citées précédemment sont mentionnés dans l'ouvrage " International Cosmetic Ingrédient Dictionnary and Handbook ", Edition 1997, pages 371 à 386 et 524 à 528, publié par "The Cosmetic, Toiletry, and Fragrance Association", dont le contenu est incorporé dans la présente demande par référence.

*Composition - charges*

La composition cosmétique peut également comporter au moins une charge, c'est-à-dire au moins une particule insoluble non colorée. Celle-ci peut être de nature minérale ou organique, de toute forme. Les charges ont pour effet de modifier la rhéologie de la composition, la texture, la sensorialité et/ou le rendu maquillage de la composition cosmétique.

On peut citer à titre d'exemple le talc, le mica, la silice, le kaolin, et les poudres de polyamide, les poudres de silicone, les poudres composites, et leurs mélanges.

Tous les composés sous forme de particules solides peuvent avoir subi un traitement de surface, notamment afin de les rendre hydrophobes ou hydrophiles. Les pigments, les filtres minéraux, et/ou les charges peuvent être enrobés après avoir subi un ou plusieurs traitements de surface de nature chimique, électronique, mécanochimique et/ou mécanique.

L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.

Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zin, de sodium, de potassium. L'acide aminé peut être par exemple la lysine, l'acide glutamique, l'alanine

Le terme alkyl mentionné dans les composés cités précédemment désigne notamment un groupe alkyle ayant de 1 à 30 atomes de carbone, de préférence ayant de 5 à 16 atomes de carbone.

*Composition - phase aqueuse*

La composition cosmétique peut en outre comprendre une ou plusieurs phases aqueuses. On entend par "phase aqueuse", une phase comprenant de l'eau et en général toute molécule à l'état dissous dans l'eau dans la composition.

La phase aqueuse des dites compositions contient de l'eau et en général d'autres solvants solubles ou miscibles dans l'eau. Les solvants solubles ou miscibles dans l'eau comprennent les mono alcools à chaîne courte par exemple en C1 -C4 comme l'éthanol, l'isopropanol; les agents humectant tels que les diols ou les polyols comme l'éthylèneglycol, le 1,2-propylèneglycol, le 1,3 -butylène glycol, l'hexylèneglycol, le diéthylèneglycol, le dipropylène glycol, et leurs mélanges.

Dans le cas d'une émulsion, simple ou multiple, la phase aqueuse peut comprendre en outre un système émulsionnant. La composition peut comprendre au moins un émulsionnant choisi parmi les ester d'acides gras de polyols comme les mono-, di-, tri- ou sesqui-oléates ou stéarates de sorbitol ou de glycérol, les laurates de glycérol ou de polyéthylène glycol ;les tensioactifs silicones comme les alkyl ou alcoxy diméthicones copolyols à chaîne alkyle ou alcoxy pendante ou en bout de squelette siliconé ayant par exemple de 6 à 22 atomes de carbone ; les polymères du type ester d'acide gras de glycol polyoxyalkyléné. La composition selon l'invention comprend un tensioactif émulsionnant permettant l'obtention d'une émulsion eau -dans -huile, notamment un tensioactif ayant un HLB (balance hydrophile/lipophile) inférieur à 7. De préférence, le tensioactif émulsionnant est choisi parmi les ester d'acides gras de polyols comme les mono-, di-, tri- ou sesqui-oléates ou stéarates de sorbitol ou de glycérol, les laurates de glycérol ou de polyéthylène glycol ; les alkyl ou alkoxy diméthicones copolyols à chaîne alkyle ou alkoxy pendante ou en bout de squelette siliconé ayant par exemple de 6 à 22 atomes de carbone ; les polymères du type ester d'acide gras de glycol polyoxyalkyléné, les alkyl ou alkoxy diméthicones copolyols à chaîne alkyle ou alkoxy pendante ou en bout de squelette siliconé ayant par exemple de 6 à 22 atomes de carbone, et leurs mélanges.

La composition peut également comprendre au moins un gélifiant de la phase aqueuse. Les principaux agents gélifiants hydrophiles utilisés sont les gélifiants polymériques réticulés et les polymères naturels. Comme gélifiants polymériques réticulés, on peut citer par exemple les polymères carboxyvinyliques, comme les produits commercialisés sous les dénominations Carbopol (nom INCI: carbomer) par la société Novéon, les polyacrylamides, les polymères dérivés de l'acide 2-acrylamido-2-méthylpropane sulfonique (AMPS) tels que le produit commercialisé par la société Clariant sous la dénomination Hostacerin AMPS, et les copolymères anioniques réticulés d'acrylamide et d'AMPS, tels que le produit commercialisé sous le nom de SEPIGEL 305 par la société Seppic. Comme polymères naturels, on peut citer par exemple les gommes de xanthane et de guar ou encore les dérivés cellulosiques, les amidons et les alginates. Ces polymères naturels sont en général utilisés en association avec les gélifiants polymériques réticulés car leur pouvoir épaississant est la plupart du temps insuffisamment important pour que l'on puisse les utiliser seuls.

*Composition - ingrédients cosmétiques usuels*

La composition cosmétique peut également comprendre des ingrédients cosmétiques usuels additionnels, choisi notamment parmi les antioxydants, les parfums, les conservateurs, les neutralisants, les vitamines, les agents hydratant, les composés auto-bronzants, les actifs anti-rides, les actifs hydrophiles ou lipophiles, les agents anti-radicaux libres, les agents déodorants, les sequestrants, et leurs mélanges.

Avantageusement, la composition cosmétique peut-être une composition de soin, une composition parfumante, ou une composition de maquillage non-labiale.

Bien entendu, les listes de composés données ne sont pas limitatives et comprennent implicitement les mélanges des différents composés cités.

L'invention pourra être mieux comprise à l'aide des exemples de réalisation non limitatifs décrits ci-après, et à l'examen du dessin annexé sur lequel :

- la figure 1 est une vue en coupe longitudinale d'un mode de réalisation de l'invention,
- la figure 2 est une vue en perspective de la grille fixée à son support du dispositif de la figure 1,
- la figure 3 est une vue partielle agrandie de la figure 2, et
- les figures 4, 5 et 6 sont des vues en coupes longitudinales respectivement du piston, du tube et du corps du dispositif de la figure 1.

Dispositif

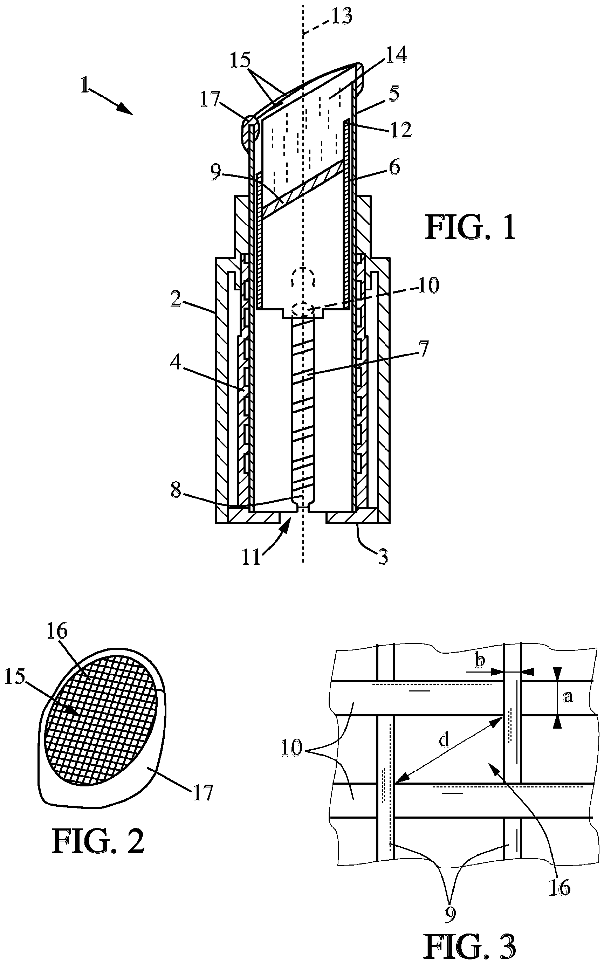

L'enveloppe extérieure du dispositif 1 représenté à la figure 1 est formée par un corps 2 et éventuellement par un capuchon (non représenté). Le capuchon peut être ôté de façon réversible. Lorsque le capuchon est en place, il vient s'emboiter sur une partie du corps, délimitée par une butée, formée par un épaulement, contre laquelle il est maintenu par encliquetage.

Le corps comprend deux portions axisymétriques, de préférence selon le même axe, et de section différente. Ces deux portions sont séparées par la butée. Le corps est creux et ouvert au niveau de ses extrémités distale et proximale, ce qui permet de ménager un logement. L'ouverture 11 de l'extrémité proximale du corps est partiellement obturée par une partie de fond 3 du corps qui s'étend, à partir de cette extrémité, dans un plan transversal au corps de l'applicateur.

Le logement ménagé dans le corps 2 comprend des moyens pour supporter et déplacer une composition cosmétique. En particulier, le logement présente avantageusement un piston 6 et un tube 5 dont les axes 13 sont confondus avec l'axe de symétrie du corps 2. Optionnellement, le logement peut comporter un élément tubulaire 4 présentant une rampe en hélice, sur sa partie la plus externe.

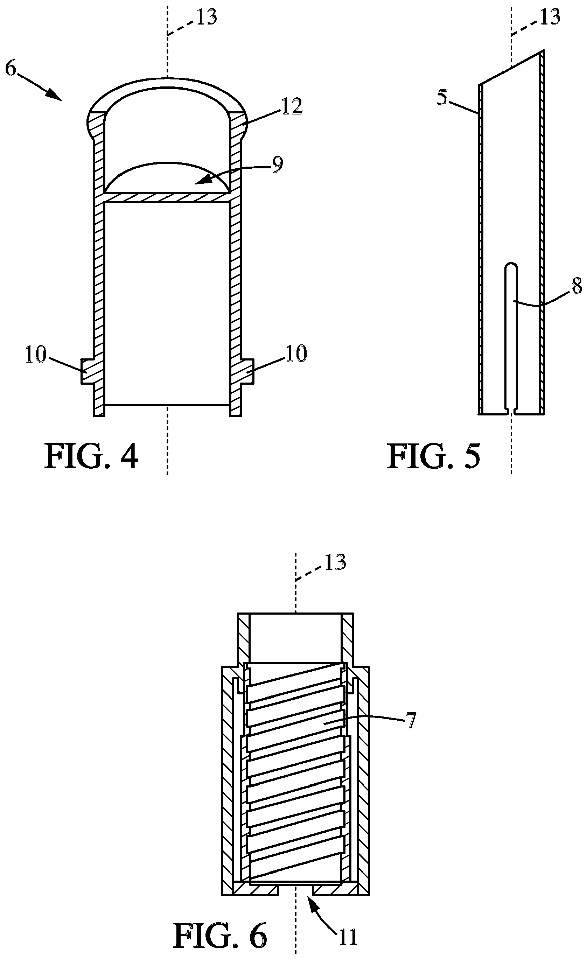

L'élément tubulaire 4 est détaillé à la figure 6. Il est creux et ouvert à ses deux extrémités. Il présente une face externe sensiblement lisse et une face interne présentant un sillon formant une rampe hélicoïdale. Cet élément repose par son extrémité proximale sur la partie de fond 3. L'extrémité distale de cet élément 4 s'étend elle sensiblement au niveau de la butée. Cet élément est rigidement fixé au corps 2.

Le tube 5 est détaillé à la figure 5 et forme un cylindre creux à section circulaire dans un plan perpendiculaire à l'axe 13 et est ouvert au niveau de ses deux extrémités. L'extrémité distale du tube 5 est par exemple biseautée. Le tube 5 peut présenter deux échancrures, comme illustré, formant deux rampes8 qui s'étendent depuis l'extrémité proximale du tube jusqu'à une portion médiane du tube. Les rampes sont de forme générale rectiligne allongée, et parallèles à l'axe 13. Elles sont sensiblement diamétralement opposées l'une à l'autre de part et d'autre de cet axe. Sur les figures 1 et 5, les deux rampes sont situées dans des plans symétriques par rapport au plan de la feuille, de sorte que seul le contour de la rampe en arrière-plan apparait puisqu'il s'agit d'une vue en coupe. Sur la figure 1, la rampe hélicoïdale 7 apparaît ainsi à travers la rampe rectiligne 8. Toutefois, dans l'exemple illustré, la rampe hélicoïdale est bien portée par l'élément tubulaire 4 et la rampe rectiligne par le tube 5.

Le piston 6 est détaillé à la figure 4 et forme un cylindre creux à section sensiblement circulaire dans un plan perpendiculaire à l'axe 13. L'extrémité proximale du piston est ouverte et son extrémité distale est en biseau et est fermée par une portion 9 qui est légèrement en retrait par rapport à l'extrémité distale du piston de façon à former une cuvette dans laquelle est engagée la base d'un bloc de composition cosmétique ou raisin. Ceci améliore la liaison entre le raisin et le piston. Sur la figure 1, la portion 9 du piston qui ferme l'extrémité distale est plane et inclinée par rapport à l'axe 13 de sorte qu'elle est aussi biseautée.

Le piston présente, au niveau de la face externe de sa portion proximale, deux plots 10 de guidage diamétralement opposés qui s'étendent en saillie de la face externe du piston. Bien entendu, on peut prévoir que les plots soient disposés à d'autres endroits de la face externe du piston. Ces plots sont aptes à parcourir chacun à la fois la rampe rectiligne 8 du tube 5 et la rampe hélicoïdale 7 de l'élément tubulaire 4. D'autres moyens de guidage du piston peuvent également convenir.

Eventuellement le piston présente en outre au niveau de son extrémité distale un bourrelet d'étanchéité 12 s'étendant sur tout le pourtour de l'extrémité distale du piston.

La portion proximale du tube 5 est donc logée à l'intérieur du corps et de l'élément tubulaire 4 et sa partie distale s'étend en dehors, en saillie du corps 2 du dispositif 1. Le tube est monté mobile à rotation autour de l'axe 13 par rapport à l'ensemble formé par le corps 2 et l'élément tubulaire 4, par des moyens de guidage adaptés. Le piston 6 est lui logé mobile à translation dans le tube 5 grâce à ses plots 10 aptes à parcourir les différentes rampes 7, 8.

Le dispositif 1 comprend en outre un bloc dur 14 de composition cosmétique ou raisin. Ce raisin 14 a une forme générale cylindrique pleine à section circulaire dans un plan perpendiculaire à l'axe 13. L'extrémité distale du raisin 14 a une forme biseautée. Bien entendu, on peut prévoir d'autres formes de cette extrémité, ces formes étant largement connues de l'homme du métier. Le raisin 14 repose par sa partie proximale sur l'extrémité distale 9 du piston 6. Le piston est donc apte à entrainer en coulissement le raisin 14 le long de l'axe 13 à l'intérieur du tube 5.

Ce raison présente une dureté supérieure à 20 grammes. La dureté est mesurée selon le protocole détaillé par la suite.

Le dispositif 1 comprend en outre une grille 15 de forme générale plane circulaire. La grille est une grille tissée en métal comprenant des fils de chaîne 18 et des fils de trame 19, ces fils formant des orifices 16 de forme générale rectangulaire. Le fait que la grille soit en métal est particulièrement avantageux puisque cela procure lors de l'application de la composition sur le corps un effet « rafraichissant » plaisant pour l'utilisateur. Avantageusement, la grille peut aussi être une toile, de préférence tissée d'un seul type de fil. L'utilisation d'une grille en matière textile permet d'obtenir de la souplesse et un meilleur confort à l'application pour l'utilisateur. Une plus grande dimension d, ici une diagonale, des orifices 16 est comprise entre 50 et 475 µm. Les fils de trame et de chaîne ont un diamètre a, b compris entre 100 et 300 µm qui correspond donc également aux valeurs de distances minimales entre les orifices. La grille 15 est rigidement fixée à l'extrémité distale du tube 5 via un support 17. Dans le présent exemple, les orifices sont comme suit :

- plus grande dimension : 280 µm,
- largeur : 100 µm, 30
- longueur : 261 µm,
- diamètre des fils de chaine a : 150 µm,
- diamètre des fils de trame b : 100 µm,
- distance entre les orifices : 100 µm.

Le support 17 a une forme générale cylindrique à section circulaire dans un plan perpendiculaire à l'axe 13. La grille 15 est par exemple associée au support 17 par surmoulage du support sur la grille, le support étant réalisée en matière plastique. Le support est lié au tube 5, cette liaison se faisant sur l'ensemble du pourtour de l'extrémité distale du tube.

Comme illustré, la ou les grilles sont par exemple orientée dans sensiblement selon un plan oblique par rapport à l'axe 13, c'est-à-dire non perpendiculaire à l'axe 13. Une telle orientation peut avantageusement permettre d'épouser la forme biseautée du raisin. On peut également prévoir que la ou les grilles sont orientées sensiblement perpendiculairement à l'axe 13.

Fonctionnement

Lorsque l'applicateur n'est pas utilisé, le piston 6 est dans sa position la plus basse, c'est-à-dire que son extrémité proximale est au contact de la partie 3 du dispositif. Le raisin 14 est situé à distance et en regard de la grille 15 (non illustré).

Lors de l'utilisation, l'utilisateur tient le dispositif 1 des deux mains, sa première main tenant l'extrémité du tube 5 faisant saillie hors du corps du dispositif et son autre main tenant le corps 2 du dispositif au niveau de sa portion de plus grande dimension.

L'utilisateur applique un mouvement de rotation au corps 2 du dispositif par rapport à au tube, autour l'axe 13. Ce mouvement de rotation entraine le parcours de la rampe hélicoïdale 7 de l'élément tubulaire 4 et de la rampe rectiligne 8 du tube 5 par les deux plots de guidage 10 du piston. Ce parcours entraine un mouvement de translation ou coulissement rectiligne du piston 6 suivant l'axe 13, le piston entrainant avec lui le raisin 14. Le mouvement de translation continue jusqu'à ce que le raisin 14 arrive au contact de la grille 15.

L'utilisateur continue d'appliquer un mouvement de rotation au corps 2 du dispositif faisant ainsi passer l'extrémité du raisin 14 à travers la grille 15. Ce passage à travers la grille entraine une déstructuration d'une fraction d'extrémité du raisin dur qui est découpée en de fines lamelles qui se réunissent ensuite après le passage de la grille, hors de celle-ci et sur celle-ci de sorte à former une pâte homogène. Une fois que le raisin a traversé la grille et qu'il a été déstructuré en pâte, cette pâte est prête à être appliquée. L'utilisateur peut ainsi aisément appliquer cette pâte sur son corps, en raison de ses propriétés d'étalement améliorées. Bien entendu, seule l'extrémité du bloc de composition cosmétique est déstructurée à chaque application, la plus grande partie du raisin gardant son intégrité. Le bloc est progressivement déstructuré au fur et à mesure des applications, tandis qu'il monte vers la grille.

Dans un mode de réalisation alternatif non représenté, le dispositif 1 comprend deux grilles 15 au niveau de l'extrémité distale du tube 5, ces deux grilles étant au contact l'une de l'autre et étant fixées sur le même support 17. Les deux grilles sont en regard l'une de l'autre et parallèles l'une à l'autre.

Lors de l'utilisation, le raisin passe à travers la grille amont puis immédiatement à travers la grille aval.

Cette disposition présente plusieurs avantages. En effet, cela permet d'avoir une déstructuration du bloc de composition cosmétique plus importante et d'obtenir ainsi une pâte plus fluide.

En outre, notamment si les plus grandes dimensions des orifices 16 de la grille proximale ou amont sont plus grandes que les plus grandes dimensions des orifices 16 de la grille distale ou aval, l'effort à fournir afin de faire traverser les grilles à une partie du raisin est réduit. Le rapport entre ces deux dimensions est supérieur à 1 et inférieur ou égal à 100, et par exemple égal à 60.

Ces deux grilles 15 peuvent être de la même matière ou dans des matières différentes. Les deux peuvent en outre avoir des orifices 16 dont les contours ont des formes différentes, que ce soit au sein d'une même grille 15 ou entre les orifices 16 des deux grilles. Par exemple, on peut prévoir une première grille dont les contours des orifices ont une forme rectangulaire et une deuxième grille dont les contours des orifices ont une forme générale oblongue. Bien entendu, un nombre plus grand de grilles peut être envisagé par exemple trois grilles, quatre grilles, cinq grilles ou six grilles.

Mesure de la dureté avant extrusion à travers la grille:

Trois composition cosmétiques, numérotées de 1 à 3 ont été préparées, et leurs duretés ont été mesurées avant et après extrusion à l'aide du dispositif cosmétique suivant l'invention.

Les compositions sont présentées dans le tableau n°1 ci-dessous, les quantités sont représentées en pourcentage par rapport au poids total de la composition. Le sigle "q.s.p" (quantité suffisante pour) signifie qu'il faut ajouter la substance en proportion suffisante pour atteindre 100% pour la formule totale.

Tableau 1: compositions cosmétiques

| Noms INCI | Composition 1 | Composition 2 | Composition 3 |
|---|---|---|---|
| SYNTHETIC WAX & ETHYLENE/PROPYLENE COPOLYMER | 11-12 | 5-5,5 | - |
| SYNTHETIC WAX & BHT | 1,5-2 | - | - |
| DIISOSTEARYL MALATE | qsp | qsp | - |
| DICAPRYLYL CARBONATE | 14-15 | - | - |
| ISONONYL ISONONANOATE | 22-23 | - | - |
| JOJOBA ESTERS & TOCOPHEROL | 4-5 | 5-5,5 | - |
| HYDROGENATED CASTOR OIL | 2-3 | - | - |
| DICAPRYLYL CARBONATE & STEARALKONIUM HECTORITE & PROPYLENE CARBONATE & TOCOPHEROL | 10-11 | - | - |
| ETHYLHEXYL PALMITATE & TRIBEHENIN & SORBITAN ISOSTEARATE & PALMITOYL-TRIPEPTIDE-1 | 3,5-4,5 | - | - |
| BUTYROSPERMUM PARKII | 2 | - | - |
| POLYMETHYLSILSESQUIOXANE | 5 | - | - |
| ALUMINUM STARCH OCTENYLSUCCINATE | 2-3 | - | - |
| DIMETHICONE (100 cSt) | 0-1 | - | - |
| HYDROGENATED COCONUT OIL | - | 16-16,5 | - |
| DIPENTAERYTHRITYL TETRAHYDROXYSTEARATE / TETRAISOSTEARATE | - | 5-5,5 | - |
| OCTYLDODECANOL | - | 8,5-9,5 | - |
| OCTYLDODECANOL & CERA ALBA (BEESWAX) | - | 9-10 | - |

(suite)

| Noms INCI | Composition 1 | Composition 2 | Composition 3 |
|---|---|---|---|
| POLYBUTENE | - | 20-22 | - |
| STEARYL HEPTANOATE & STEARYL CAPRYLATE | - | 5,5-6,5 | - |
| C20-24 ALKYL DIMETHICONE | - | 0-0,5 | - |
| POLYETHYLENE | - | 2-2,5 | - |
| OCTYLDODECANOL & DISTEARDIMONIUM HECTORITE & PROPYLENE CARBONATE | - | 6,5-7 | - |
| PHYTOSTERYL/OCTYLDODECYL LAUROYL GLUTAMATE | - | 2-2,5 | - |
| CETYL PHOSPHATE | - | - | 1,5 |
| GLYCERIN | - | - | 10 |
| AQUA (WATER) | - | - | 10 |
| ARGININE | - | - | 0,75 |
| DICAPRYLYL CARBONATE | - | - | 17,14 |
| CANDELILLA CERA (EUPHORBIA CERIFERA (CANDELILLA) WAX) & BENZYL ALCOHOL | - | - | 8 |
| JOJOBA ESTERS & POLYGLYCERIN-3 & ACACIA DECURRENS FLOWER WAX & HELIANTHUS ANNUUS CERA SEED (HELIANTHUS ANNUUS (SUNFLOWER) SEED WAX) | - | - | 8 |
| CERA ALBA (BEESWAX) | - | - | 4 |
| SUCROSE TETRASTEARATE TRIACETATE | - | - | 2 |
| BORON NITRIDE | - | - | 1 |
| ETHYLHEXYL METHOXYCINNAMATE & BHT | - | - | 5 |
| SQUALANE | - | - | 6,14 |
| TITANIUM DIOXIDE & STEARIC ACID & ALUMINA & SILICA | - | - | 7 |
| ZINC OXIDE & TRIETHOXYCAPRYLYLSILANE | - | - | 2 |
| DICAPRYLYL CARBONATE | - | - | 6,67 |
| Pigments | 6,5-7,5 | 2-4 | 10,4 |
| conservateurs | 0,5 | 0,5 | 0,4 |
| parfums | 0,08 | 0,1 | - |
| TOTAL | 100 | 100 | 100 |

Les compositions 1 à 3 sont chauffées de manière à ce qu'elles se trouvent totalement à l'état liquide. Les compositions 1 à 3 du tableau n°1 sont chacune coulées à chaud dans trois contenants, en l'espèce trois pots en verre à parois circulaires et à fond plat et rond, de diamètre intérieur 30 mm ±0,3 mm. Trois échantillons par composition sont alors préparés en introduisant une quantité de composition cosmétique suffisante pour obtenir une épaisseur de composition comprise entre 8 et 9 mm dans le contenant. Les échantillons sont conservés à 20°C pendant une durée supérieure à 24h avant d'être caractérisés.

La dureté de chaque échantillon est évaluée à 20°C à l'aide d'un texturomètre TA-XT Plus Microstable System de la société Swantech. Un mobile de type cylindrique en inox d'un diamètre de 2 mm pénètre dans chaque échantillon à une vitesse de 1 mm.s-1 jusqu'à une profondeur de 3 mm, puis retourne à sa position initiale après chaque mesure. Quatre mesures sont réalisées sur chacun des trois échantillons - soit douze mesures au total-en faisant pénétrer le mobile à différents endroits.

La valeur de dureté retenue pour chaque composition correspond à la moyenne des douze mesures effectuées sur les trois échantillons. Elle est notée d1 et est exprimée en grammes (g). Les valeurs sont reportées dans le tableau n°2.

Mesure de la dureté après extrusion à travers la grille:

La préparation des échantillons pour la mesure de dureté après extrusion à travers une grille du dispositif cosmétique selon l'invention se fait en deux temps.

Dans un premier temps, les compositions 1 à 3 sont chauffées de sorte qu'elles se trouvent totalement à l'état liquide, et coulées à chaud dans le réservoir d'un article cosmétique correspondant à un exemple de mise en œuvre du dispositif selon l'invention, muni de deux grilles. La première grille en amont de la sortie de la composition cosmétique est constituée d'une toile tissée d'un seul type de fils ayant un diamètre de 112 $\mu$m et des ouvertures de 160 $\mu$m en moyenne. La seconde grille en aval de la sortie de la composition cosmétique est constituée d'une toile tissée d'un seul type de fils, différents de la première grille, ayant un diamètre de 36 $\mu$m et des ouvertures de 75 $\mu$m en moyenne. Un seul échantillon par composition est préparé, et il est conservé pendant une durée supérieure à 24h à 20°C avant d'être caractérisé.

Les compositions sont ensuite extrudées à travers les deux grilles successives du dispositif cosmétique, puis transférées dans des pots en verre à parois circulaires et à fond plat et rond, de diamètre intérieur 30 mm ± 0,3 mm. La surface de chaque échantillon est lissée à l'aide d'un emporte-pièce présentant une surface lisse et ronde de diamètre 30 mm.

La dureté de chaque échantillon est évaluée à 20°C à l'aide d'un texturomètre TA-XT Plus Microstable System de la société Swantech. Un mobile de type cylindrique en inox d'un diamètre de 2 mm pénètre dans chaque échantillon à une vitesse de 1 mm.s-1 jusqu'à une profondeur de 3 mm, puis retourne à sa position initiale après chaque mesure. Quatre mesures sont réalisées par échantillon, en faisant pénétrer la sonde à différents endroits pour chacune de ces quatre mesures.

La valeur de dureté retenue pour chacune des compositions 1 à 3 correspond à la moyenne des quatre mesures effectuées par échantillon. Elle est notée d2 et est exprimée en grammes (g). Les valeurs sont reportées dans le tableau n°2.

La variation de la dureté de chaque composition avant et après passage à travers les grilles est notée $\Delta d$ et exprimée en pourcentage (%). Elle est obtenue par la formule suivante:

$$\Delta d = \frac{(d2 \times 100)}{d1} - 100$$

Tableau 2: valeurs de dureté et de variations de la dureté après extrusion à travers un premier exemple de mise en oeuvre de l'invention

|  | d1 (g) | d2 (g) | $\Delta d$ (%) |
|---|---|---|---|
| **Composition 1** | 246,8 | 15 | - 93,9 |
| **Composition 2** | 111,7 | 2,4 | - 97,8 |
| **Composition 3** | 21,4 | 5,8 | - 72,9 |

Les valeurs négatives de $\Delta d$ indiquent que la composition subit une réduction de sa dureté après extrusion.

Les trois compositions permettent de couvrir une large gamme de compositions cosmétiques de type solide ou semi-solide. Ces résultats mettent en évidence que cet exemple de mise en œuvre du dispositif cosmétique permet d'obtenir une réduction de la dureté d'au moins 73%.

La dureté de la composition n°3, qui présente la plus faible dureté avant extrusion (dl), est à nouveau mesurée après extrusion à travers un autre jeu de grilles afin de mesurer l'impact de la taille des ouvertures au niveau de la toile constituant la grille. De la même façon que précédemment, la composition 3 est coulée à chaud dans le réservoir d'un dispositif cosmétique selon l'invention, muni d'une première grille en amont de la sortie de la composition cosmétique formée d'une toile tissée d'un seul type de fils ayant un diamètre de 236 $\mu$m et des ouvertures de 475 $\mu$m. . La seconde grille en aval de la sortie de composition cosmétique est constituée d'une toile tissée d'un seul type de fils, différents de la première grille, ayant un diamètre de 90 $\mu$m et des ouvertures de 125 $\mu$m en moyenne Les valeurs de la dureté après extrusion à travers le dispositif, notée d2' ainsi que la variation de dureté après extrusion à travers cette variante du dispositif cosmétique, $\Delta d'$ sont données dans le tableau n°3.

Tableau 3: valeurs de dureté et de variations de la dureté après extrusion à travers un deuxième exemple de mise en œuvre de l'invention

| | d1 (g) | d2' (g) | Δd'(%) |
|---|---|---|---|
| **Composition 3** | 21,4 | 6,6 | -69,1 |

Le deuxième exemple de mise en œuvre d'un dispositif selon l'invention permet une réduction de la dureté de la composition n°3 après extrusion de 70% ce qui reste satisfaisant.

A moins que le contraire ne soit précisé, le mot « ou » est équivalent à « et/ou ». De même, le mot « comprends un » est équivalent à « comprends, entre autre, au moins un » sauf si le contraire est précisé.

**Revendications**

1.  Dispositif (1) de conditionnement et de distribution d'une composition cosmétique comprenant :

    - un logement recevant une composition cosmétique présentant une dureté (d) supérieure ou égale à 20g,
    - au moins une grille en métal agencée pour le passage de la composition cosmétique à travers la grille et présentant des orifices traversants,
    le dispositif étant configuré de sorte que la composition cosmétique subisse une réduction de sa dureté (Δd) supérieure à 50%, de préférence supérieure à 70%, lors du passage de la composition cosmétique à travers ladite au moins une grille, les orifices traversants présentant une plus grande dimension comprise entre 50 et 475 μm,

    et dans lequel la grille ou au moins une des grilles est tissée.

2.  Dispositif selon l'une quelconque des revendications précédentes comprenant au moins deux grilles se faisant suite suivant une direction de passage de la composition cosmétique à travers les grilles.

3.  Dispositif selon l'une quelconque des revendications précédentes, la composition cosmétique se présentant sous forme anhydre.

4.  Dispositif selon l'une quelconque des revendications 1 à 2, la composition cosmétique se présentant sous la forme d'une émulsion, de préférence d'une émulsion multiple.

5.  Dispositif selon la revendication précédente, l'émulsion étant une émulsion huile dans eau.

6.  Dispositif selon la revendication 2, l'émulsion étant une émulsion eau dans huile.

7.  Dispositif selon l'une quelconque des revendications précédentes, la composition cosmétique étant conditionnée sous une forme choisie parmi une composition coulée, un gel aqueux et un gel huileux.

8.  Dispositif selon l'une quelconque des revendications précédentes, la composition comprenant 0,5 à 90% en poids de composés gras structurants choisis parmi des cires, de préférence des cires présentant un bas point de fusion, des corps gras pâteux, des argiles rendues hydrophobes, et des composés siliconés.

9.  Dispositif selon l'une quelconque des revendications précédentes, la composition comprenant 0,01 à 80% en poids d'huiles liquides à 25°C choisies parmi des huiles hydrocarbonées et/ou siliconées.

10. Dispositif selon l'une quelconque des revendications précédentes, la composition comprenant au moins un filmogène choisi parmi les filmogènes hydrocarbonés et/ou siliconés.

11. Dispositif selon l'une quelconque des revendications précédentes, la composition comprenant 0 à 50 % en poids d'au moins un filtre ultraviolet.

12. Dispositif selon l'une quelconque des revendications précédentes, la composition comprenant 0 à 50% en poids d'au moins un pigment de préférence choisi parmi le dioxyde de titane, les oxydes de fer, le carmin de cochenille, les laques organiques.

13. Dispositif selon l'une quelconque des revendications précédentes, la composition comprenant 0 à 50% en poids d'au moins un pigment interférentiel, comprenant de préférence du mica, de la silice, du verre et/ou du dioxyde de titane.

14. Dispositif selon l'une quelconque des revendications précédentes, la composition cosmétique étant une composition de soin, une composition parfumante, ou une composition de maquillage non-labiale.


**Patentansprüche**

1. Vorrichtung (1) zum Verpacken und Abgeben einer kosmetischen Zusammensetzung, umfassend:

   - ein Gehäuse, das eine kosmetische Zusammensetzung mit einer Härte (d) größer oder gleich 20 g aufnimmt,
   - wenigstens ein Metallgitter, das für den Durchlass der kosmetischen Zusammensetzung durch das Gitter angeordnet ist und Durchgangslöcher aufweist,

   wobei die Vorrichtung derart ausgebildet ist, dass die kosmetische Zusammensetzung eine Verringerung ihrer Härte ($\Delta$d) um mehr als 50 %, bevorzugt mehr als 70 %, erfährt, wenn die kosmetische Zusammensetzung das wenigstens eine Gitter durchläuft, wobei die Durchgangslöcher eine größte Abmessung zwischen 50 und 475 $\mu$m aufweisen, und wobei das Gitter oder wenigstens eines der Gitter gewebt ist.

2. Vorrichtung nach einem der vorhergehenden Ansprüche, umfassend wenigstens zwei Gitter, die entlang einer Durchlaufrichtung der kosmetischen Zusammensetzung durch die Gitter aufeinander folgen.

3. Vorrichtung nach einem der vorhergehenden Ansprüche, wobei die kosmetische Zusammensetzung in wasserfreier Form vorliegt.

4. Vorrichtung nach einem der Ansprüche 1 bis 2, wobei die kosmetische Zusammensetzung in Form einer Emulsion, bevorzugt einer Mehrfachemulsion, vorliegt.

5. Vorrichtung nach dem vorhergehenden Anspruch, wobei die Emulsion eine Öl-in-Wasser-Emulsion ist.

6. Vorrichtung nach Anspruch 2, wobei die Emulsion eine Wasser-in-Öl-Emulsion ist.

7. Vorrichtung nach einem der vorhergehenden Ansprüche, wobei die kosmetische Zusammensetzung in einer Form verpackt ist, die aus einer gegossenen Zusammensetzung, einem wässrigen Gel und einem öligen Gel gewählt ist.

8. Vorrichtung nach einem der vorhergehenden Ansprüche, wobei die Zusammensetzung 0,5-90 Gew.-% fettstrukturierende Verbindungen aufweist, die aus Wachsen, bevorzugt Wachsen mit einem niedrigen Schmelzpunkt, pastösen Fettkörpern, hydrophobierten Tonerden und Silikonverbindungen gewählt sind.

9. Vorrichtung nach einem der vorhergehenden Ansprüche, wobei die Zusammensetzung bei 25°C 0,01-80 Gew.-% flüssige Öle, gewählt aus Kohlenwasserstoff- und/oder Silikonölen, aufweist.

10. Vorrichtung nach einem der vorhergehenden Ansprüche, wobei die Zusammensetzung wenigstens einen Filmbildner aufweist, der aus Kohlenwasserstoff- und/oder Silikonfilmbildnern gewählt ist.

11. Vorrichtung nach einem der vorhergehenden Ansprüche, wobei die Zusammensetzung 0-50 Gew.-% wenigstens eines Ultraviolettfilters aufweist.

12. Vorrichtung nach einem der vorhergehenden Ansprüche, wobei die Zusammensetzung 0 bis 50 Gew.-% wenigstens eines Pigments aufweist, das bevorzugt aus Titandioxid, Eisenoxiden, Cochenille-Karmin und organischen Lacken gewählt ist.

**13.** Vorrichtung nach einem der vorhergehenden Ansprüche, wobei die Zusammensetzung 0-50 Gew.-% wenigstens eines Interferenzpigments aufweist, das bevorzugt Glimmer, Siliciumdioxid, Glas und/oder Titandioxid umfasst.

**14.** Vorrichtung nach einem der vorhergehenden Ansprüche, wobei die kosmetische Zusammensetzung eine Hautpflegezusammensetzung, eine Parfümzusammensetzung oder eine nicht-labiale Make-up-Zusammensetzung ist.

**Claims**

**1.** A device (1) for packaging and dispensing a cosmetic composition comprising:

- a housing receiving a cosmetic composition having a hardness (d) greater than or equal to 20g,
- at least one metal mesh arranged for the passage of the cosmetic composition through the mesh and having through orifices,

the device being configured such that the cosmetic composition undergoes a reduction in its hardness ($\Delta d$) greater than 50%, preferably greater than 70%, during the passage of the cosmetic composition through said at least one mesh, the through orifices having a larger dimension comprised between 50 and 475 $\mu$m, and wherein the mesh or at least one of the meshes is woven.

**2.** The device according to any one of the preceding claims, comprising at least two meshes being in succession in a direction of passage of the cosmetic composition through the meshes.

**3.** The device according to any one of the preceding claims, the cosmetic composition being in anhydrous form.

**4.** The device according to any one of claims 1 to 2, the cosmetic composition being in the form of an emulsion, preferably of a multiple emulsion.

**5.** The device according to the preceding claim, the emulsion being an oil-in-water emulsion.

**6.** The device according to claim 2, the emulsion being a water-in-oil emulsion.

**7.** The device according to any one of the preceding claims, the cosmetic composition being packaged in a form selected from a cast composition, an aqueous gel and an oily gel.

**8.** The device according to any one of the preceding claims, the composition comprising 0.5 to 90% by weight of structuring fatty compounds selected from waxes, preferably waxes having a low melting point, pasty fatty substances, hydrophobised clays, and silicone compounds.

**9.** The device according to any one of the preceding claims, the composition comprising 0.01 to 80% by weight of liquid oils at 25°C selected from hydrocarbon and/or silicone oils.

**10.** The device according to any one of the preceding claims, the composition comprising at least one film former selected from the hydrocarbon and/or silicone film formers.

**11.** The device according to any one of the preceding claims, the composition comprising 0 to 50% by weight of at least one ultraviolet filter.

**12.** The device according to any one of the preceding claims, the composition comprising 0 to 50% by weight of at least one pigment preferably selected from titanium dioxide, iron oxides, cochineal carmine, organic lacquers.

**13.** The device according to any one of the preceding claims, the composition comprising 0 to 50% by weight of at least one interference pigment, preferably comprising mica, silica, glass and/or titanium dioxide.

**14.** The device according to any one of the preceding claims, the cosmetic composition being a care composition, a perfume composition, or a non-lip makeup composition.

FIG. 1

FIG. 2

FIG. 3

**FIG. 4**

**FIG. 5**

**FIG. 6**

## RÉFÉRENCES CITÉES DANS LA DESCRIPTION

**Documents brevets cités dans la description**

- EP 1072214 A

- FR 2933865

**Littérature non-brevet citée dans la description**

- International Cosmetic Ingrédient Dictionnary and Handbook. 1997, 371-386, 524-528